# EUROPEAN PATENT APPLICATION

(11) **EP 4 567 820 A2**
(43) Date of publication of application: **11.06.2025**
(21) Application number: 24216764.1
(22) Date of filing: 02.12.2024
(51) Int. Cl.: G16H 10/40

(54) **BUILDING AND EXECUTING A MEDICAL ALGORITHM**

(30) Priority: 05.12.2023 US 202318528941; 11.01.2024 LU 103235
(71) Applicant: Instrumentation Laboratory Company, Bedford, MA 01730 (US)
(72) Inventor: AVILA, John, Bedford, 01730 (US); COCKERELL, Jullian, Bedford, 01730 (US); GOERLINGER, Klaus, Bedford, 01730 (US); HAGMANN, Anna, Bedford, 01730 (US); KHUNGER, Sanjay, Bedford, 01730 (US); KIM, David, Bedford, 01730 (US); LI, Haisong, Bedford, 01730 (US); TOMI, Nils, Bedford, 01730 (US); WINKLER, Anne, Bedford, 01730 (US); JOHNSON, Soren, Bedford, 01730 (US)
(74) Representative: Müller-Boré & Partner Patentanwälte PartG mbB

(57) **Abstract**

In an example, one or more non-transitory machine-readable storage media store instructions that are executable by one or more processing devices to perform a method that includes: outputting a user interface configured to receive inputs to use in a medical algorithm; configuring the medical algorithm based on the inputs; and outputting a display. The display includes a representation of the medical algorithm. The representation presents operations to be executed by the medical algorithm. The representation relates the operations to one or more notifications triggered based on the operations.

## Description

### TECHNICAL FIELD

This specification relates generally to systems and processes for building and executing a medical algorithm.

### BACKGROUND

An example test instrument is configured to generate parameters based on assays performed on a test sample. For example, a viscoelastic test instrument may be configured to receive a test sample, such as whole blood or a component or derivative thereof, and to perform one or more assays to generate one or more parameters based, for example, on clotting that occurs in the test sample. The one or more parameters may be analyzed to detect a pathology (e.g., low fibrin contribution to clot firmness), to diagnose a condition of the patient (e.g., hypofibrinogenemia), and/or to suggest a treatment for the condition (e.g., cryoprecipitate transfusion).

### SUMMARY

In an example, one or more non-transitory machine-readable storage media store instructions that are executable by one or more processing devices to perform a method that includes: outputting a user interface configured to receive inputs to use in a medical algorithm; configuring the medical algorithm based on the inputs; and outputting a display. The display includes a representation of the medical algorithm. The representation presents operations to be executed by the medical algorithm. The representation relates the operations to one or more notifications triggered based on the operations. The one or more non-transitory machine-readable storage media may include one or more of the following features, either alone or in combination.

The inputs may include one or more parameters, one or more predefined values, one or more operators, and the one or more notifications. The operations may include one or more rules. A rule may be configured to evaluate a test result value for one of the parameters against one of the predefined values based on one of the operators. The notifications may correspond to the operations.

The one or more parameters may include one or more of the following: a parameter associated with viscoelastic testing, a parameter associated with blood gas testing, a parameter associated with platelet function testing, a parameter associated with coagulation testing, or a parameter associated with activated clotting time (ACT) testing. The medical algorithm may be for use with a patient. The inputs may include information about at least one of (i) whether the patient is bleeding or has non-bleeding, thrombosis, (ii) a clinical setting, (iii) a stage of surgery the patient is undergoing, (iv) a medical condition of the patient, (v) a body weight of the patient, (vi) an age of the patient, (vii) a sex of the patient, (viii) a medical diagnosis for the patient, or (ix) a treatment that the patient has undergone or is undergoing.

The operations may include one or more groups. A group may include one or more steps. The group may be executable independently of others of the groups.

The operations may include multiple groups. Each group may include multiple steps. Within a group among the multiple groups, the steps may be executable sequentially. Steps in a group among the multiple groups may be executable independently of steps in other groups among the multiple groups.

The display may include an element that is selectable to verify the medical algorithm. The element may be configured to cause output of the instruction.

The inputs may include parameters, predefined values, and operators. The method may include receiving, from the display, an instruction corresponding to an action to take with respect to the medical algorithm. The operations may include rules configured to evaluate test result values for parameters against respective predefined values based on respective operators. The action may include verifying the medial algorithm and, in response to the instruction, the method performed by the one or more processing devices may include outputting a second user interface configured to receive mock or prior test result values for the parameters. The method performed by the one or more processing devices may include: executing the medical algorithm using the mock or prior test result values received via the second user interface; and outputting a report representing one or more results produced by executing the medical algorithm using the or prior mock test result values. The report may include one or more of the rules, one or more test result values received via the second user interface for the one or more rules, and at least one of: (i) a time associated with execution of the one or more rules, (ii) a notification triggered by execution of the one or more rules, or (iii) an alert triggered by execution of the one or more rules, the alert being indicative of one or more test result values satisfying the one or more rules.

In an example, one or more non-transitory machine-readable storage media store instructions that are executable by one or more processing devices to perform a method that includes: receiving test result values for parameters associated with one or more assays; and executing a medical algorithm using the test result values. The medical algorithm includes rules configured to evaluate the test result values. The medical algorithm includes groups including one or more steps, with each step including one or more of the rules. Executing the medical algorithm includes: when a group is identified during execution of the medical algorithm, executing the group independently of other groups in the medical algorithm; when a series of steps is identified in a group during execution of the medical algorithm, executing the steps in the series in a sequence in which the steps are arranged in the series; and outputting a report representing results produced by executing the medical algorithm. The report includes the rules, values based on test result values associated with corresponding rules, and at least one of: (i) a time associated with execution of the rules, (ii) a notification triggered by execution of the one or more rules, or (iii) an alert triggered by execution one or more of the rules. The alert is indicative of one or more test result values satisfying the one or more rules. The one or more non-transitory machine-readable storage media may include one or more of the following features, either alone or in combination.

The values based on the test result values may include at least one of (i) a test result value, (ii) a maximum value that is less than a corresponding test result value, or (iii) a minimum value that is greater than a corresponding test result value. The test result values may be received from a viscoelastic testing system. The test result values may be received in metadata of one or more images or received from an optical character recognition of the one or more images. The method may be performed by the one or more processing devices may include displaying the report together with the one or more images. At least one of the images may include a reaction curve that shows an elasticity over time when a blood clot forms or dissolves. A test result values for a parameter may include (i) a final value for the parameter or (ii) a value for the parameter that is non-final. The medical algorithm may be for use with a patient. The method performed by the one or more processing devices may include receiving information about at least one of (i) whether the patient is bleeding, (ii) a type of surgery that the patient is undergoing, (iii) a stage of the surgery, or (iv) a medical condition of the patient. The medical algorithm may be executed based on the information.

An example system includes one or more test instruments configured to produce test result values for parameters associated with one or more assays; and one or more processing devices in communication with the one or more test instruments. The one or more processing devices are configured to execute instructions to perform operations that include: receiving the test result values from the one or more test instruments; and executing a medical algorithm using the test result values. The medical algorithm may include rules configured to evaluate the test result values. The medical algorithm may include groups including one or more steps, with each step including one or more of the rules. Executing the medical algorithm includes: when a group is identified during execution of the medical algorithm, executing the group independently of other groups in the medical algorithm; when a series of steps is identified in a group during execution of the medical algorithm, executing the steps in the series in a sequence in which the steps are arranged in the series; and outputting a report representing results produced by executing the medical algorithm. The report may include the rules, values based on test result values associated with corresponding rules, and at least one of: (i) a time associated with execution of the rules, (ii) a notification triggered by execution of the one or more rules, or (iii) an alert triggered by execution one or more of the rules The alert is indicative of one or more test result values satisfying the one or more rules.

The one or more processing devices may be external to the one or more test instruments. The one or more processing devices may be internal to one or more of the test instruments.

At least part of the devices, systems, and processes described in this specification may be executed on, or controlled by, executing on one or more processing devices instructions that are stored on one or more non-transitory machine-readable storage media. Examples of non-transitory machine-readable storage media include read-only memory, an optical disk drive, memory disk drive, and random access memory (RAM). At least part of the devices, systems, and processes described in this specification may be implemented or controlled using a computing system comprised of one or more processing devices and memory storing instructions that are executable by the one or more processing devices to perform various control operations. The devices, systems, and processes described in this specification may be configured, for example, through design, construction, formulation, arrangement, placement, programming, operation, activation, deactivation, and/or control.

Two or more of the features described in this specification, including in this summary section, may be combined to form implementations not specifically described in this specification.

The details of one or more implementations are set forth in the accompanying drawings and the description below. Other features and advantages will be apparent from the description and drawings, and from the claims.

### DESCRIPTION OF THE DRAWINGS

Figs 1 to 9 are examples of a user interface (Ul) for building a medical algorithm.
Fig. 10 is an example of UI showing comments on a displayed part of the medical algorithm.
Fig. 11 is an example of a UI for providing mock or prior test result values to be evaluated by the medical algorithm to verify the medical algorithm.
Fig. 12 is an example of a UI for providing a report based on the mock or prior test result values.
Fig. 13 is a flowchart showing example operations included in an example process for building and verifying the medical algorithm.
Fig. 14 is a flowchart showing example operations included in an example process for executing the medical algorithm.
Fig. 15 is an example of a report generated by the medical algorithm.
Fig. 16 is an example of a report generated by the medical algorithm displayed alongside images containing reaction curves.
Fig. 17 is a block diagram of an example test system configured to build, to verify, and to execute the medical algorithm.

Like reference numerals indicate like elements.

### DETAILED DESCRIPTION

Described herein are systems and processes for generating user interfaces that enable a user to provide information for one or more medical algorithms and notifications therefor, and for automatically generating and executing the medical algorithm based on the information using test result values.

The medical algorithm(s) include executable operations to analyze the test result values. The test result values may be for one or more parameters obtained by performing one or more assays on one or more test samples. The medical algorithm may be built by organizing the executable operations into groups, steps, and rules.

Examples of types of the parameters may include, but are not limited to, one or more parameters associated with viscoelastic testing, one or more parameters associated with blood gas testing, one or more parameters associated with platelet function testing, one or more parameters associated with coagulation testing, and/or one or more parameters associated with activated clotting time (ACT) testing.

Test result values for the parameters may be obtained by performing one or more assays on one or more test instruments. Examples of viscoelastic test instruments that may perform one or more assays to generate test result values for the parameters include, but are not limited to, the ROTEM^{®} *sigma* system or the ROTEM^{®} *delta* system by Werfen^{®}, the TEG^{®} 6s and ClotPro^{®} system by Haemonetics^{®}, and the QUANTRA^{®} system by HemoSonics^{®}. Examples of the parameters for which test result values may be generated by these example viscoelastic test instruments include, but are not limited to, the following clot initiation parameters, clot kinetic parameters, clot firmness parameters, and clot lysis parameters.

| CATEGORY | ROTEM^{®} and ClotPro^{®} | TEG^{®} | QUANTRA^{®} |
|---|---|---|---|
| Clot initiation parameter | CT | CT, R-time | CT; CTH; CTR |
| Clot kinetics parameter | CFT; alpha angle | K-time; alpha angle | |
| Clot firmness parameter | A5; A10; A20; MCF | A5; A10; MA | CS; PCS; FCS |
| Clot lysis parameter | ML; LI30; LI45; LI60; LOT; LT | LY30; LY60; ML | CSL |

For ROTEM^{®} and CloPro^{®}, CT refers to clotting time; CFT refers to clot formation time, the alpha angle refers to the angle tangent to the clotting curve at a two millimeter (mm) amplitude point, A5 refers to clot firmness amplitude five minutes after CT; A10 refers to clot firmness amplitude ten minutes after CT; A20 refers to clot firmness twenty minutes after CT; MCF refers to maximum clot firmness; ML refers to maximum lysis; LI30 refers to a lysis index thirty minutes after CT; LI45 refers to a lysis index 45 minutes after CT; LI60 refers to a lysis index 60 minutes after CT; LOT refers to lysis onset time; and LT refers to lysis time.

For TEG^{®}, CT refers to clotting time, R-time refers to reaction time; K-time refers to kinetic time, the alpha angle refers to the speed of fibrin accumulation and polymerization and is closely related to K-time; A5 refers to clot firmness amplitude five minutes after CT; A10 refers to clot firmness amplitude ten minutes after CT; MA refers to maximum amplitude; ML refers to maximum lysis; LY30 refers to lysis 30 minutes after MA; and LY60 refers to lysis 60 minutes after MA.

For Quantra^{®}, CT refers to clotting time without heparinase; CTH refers to clotting time with heparinase, CTR refers to clotting time ratio; CS refers to clot stiffness; PCS refers to platelet contribution to clot stiffness; FCS refers to fibrinogen contribution to clot stiffness; and CSL refers to clot stability to lysis.

The parameters may be obtained using an activated clotting time (ACT) test performed on the GEM^{®} Hemochron^{™} 100 system.

Standard coagulation tests may include D-dimer tests, prothrombin time (PT) tests, international normalized ratio (INR) blood tests, activated partial thromboplastin clotting time (aPTT) tests, thrombin time (TT) tests, diluted thrombin time (dTT) tests, calibrated direct oral anticoagulants (DOAC) tests, platelet function tests, and platelet count tests. The parameters may be obtained from one or more of these tests.

Platelet function tests may include an aspirin test and a P2Y12 reactions units (PRU) test performed on the VerifyNow^{™} system. Platelet function tests may include ROTEM^{®} platelet tests, multiplate tests, TEGO platelet mapping tests, and platelet function analyzer (PFA) 100/200 tests. The parameters may be obtained from one or more of these tests.

The parameters may include other variables such as patient temperature and trauma scores such as ABC trauma scores or trauma-associated severe hemorrhage trauma scores. The parameters may identify a clinical condition of a patient, such as bleeding, non-bleeding, or thrombosis, and a clinical setting, such as cardiovascular surgery, trauma, orthopedic surgery, liver surgery, transplantation, or obstetrics.

The parameters may be obtained from a blood gas testing. Examples of parameters from a blood gas testing system, such as the GEM^{©} Premier 500 blood gas testing system, that may be used may include, but are not limited to, BE(B) (base excess), BE(ecf) (extracellular base excess), tHb(c) (total hemoglobin), Ca++ (7.4) (ionized calcium with a pH of 7.4), AG (anion gap) P/F ratio (pressure of oxygen in arterial blood (PaO₂) to the fraction of inspiratory oxygen concentration (FiO₂)), pAO₂ (partial pressure of oxygen), CaO₂ (arterial oxygen content), CvO₂ (central venous oxygen saturation), p50 (hemoglobin-O₂ affinity), O₂cap (arterial blood gas), sO2(c) (oxygen saturation), O₂ct (oxygen content), HCO3 - std (amount of bicarbonate in blood in a form of carbon dioxide), TCO₂ (total blood CO₂) HCOs - (c) (bicarbonate, a measure of acid-base balance), A-aDO₂ (alveolar-arterial oxygen gradient), paO₂/pAO₂ (partial pressure of oxygen divided by partial pressure of carbon dioxide, RI (resistive index, an ultrasound parameter), CcO₂ (capillary oxygen content), a-vDO2 (arteriovenous difference of oxygen content), Qsp/Qt (est) (intrapulmonary shunting estimated), Qsp/Qt (intrapulmonary shunting), and Hct(c) (hematocrit).

Examples of other parameters that may be used are listed in U.S. Patent Publication No. 2016/0161510 titled "Blood Testing System Result Interpreter Interface and Methods", the contents of which are incorporated herein by reference.

An example rule in the medical algorithm includes an operation to evaluate a test result value for a parameter from an assay against a single predefined value or to evaluate a value that is based on multiple test result values for combinations of multiple parameters against a single predefined value. The evaluation may be performed using a mathematical operator, such as "=", ">", "<", "≥", "≤", and so forth

An example step in the medical algorithm includes a single rule, or multiple rules related by a conjunction, such as "AND" or "OR", that direct(s) an outcome, referred to as a notification, such as a summary, a diagnosis, and/or a treatment option. The notification may be displayed along with an alert. An alert indicates that the rules in the step were satisfied, as described in more detail below.

An example group in the medical algorithm includes a single step or a sequence of multiple steps that may be executed independently of one, more than one, or all other groups in the medical algorithm. For example, a medical professional may determine that certain steps are required to provide diagnosis and/or treatment options for a particular condition, and that those steps must be performed in a certain, predetermined order and associated with specific clinical conditions and specific clinical settings. Accordingly, those steps are assigned by the algorithm creator to a single group and, when executed within that group, are executed in sequence. Independent execution may include that at least part of the steps in two or more groups execute at the same time. Independent execution may include that at least part of the steps in two or more groups execute within the same time period, but not necessarily at the same time.

Treatment options, in particular, may only make sense in combination with specific clinical condition, such as bleeding or thrombosis, and/or specific clinical settings, such as cardiovascular surgery, trauma, orthopedic surgery, liver surgery, transplantation, or obstetrics. Accordingly, parameters relating to the clinical conditions and clinical settings may also be incorporated into the medical algorithm.

By executing each group independently from other groups in the medical algorithm, groups that receive parameters that require less time for evaluation can be executed sooner than groups that receive parameters that require more time for evaluation. The medical algorithm may provide results from groups as the groups complete execution. In some instances, one or more parameters that be assessed more quickly (which can be organized in a first group) by the medical algorithm, which can predict a condition that is later confirmed by other parameters (which are grouped in a second group). For example, the clotting time (CT) and 5 min clot firmness parameter (A5) (e.g., in a first group) occur before parameters like the lysis index at 30 min (LI30) (e.g., in a second group) that predict delayed fibrinolysis. This may be important because delayed treatment may not be effective. For example, tranexamic acid only has a benefit within about three hours of the bleeding onset. Since unavoidable delays of transporting a patient and testing occur, there may be little time to react. It can be better to prophylactically administer the drug in response to analysis of parameters in the first group, rather than waiting for all other groups to be analyzed.

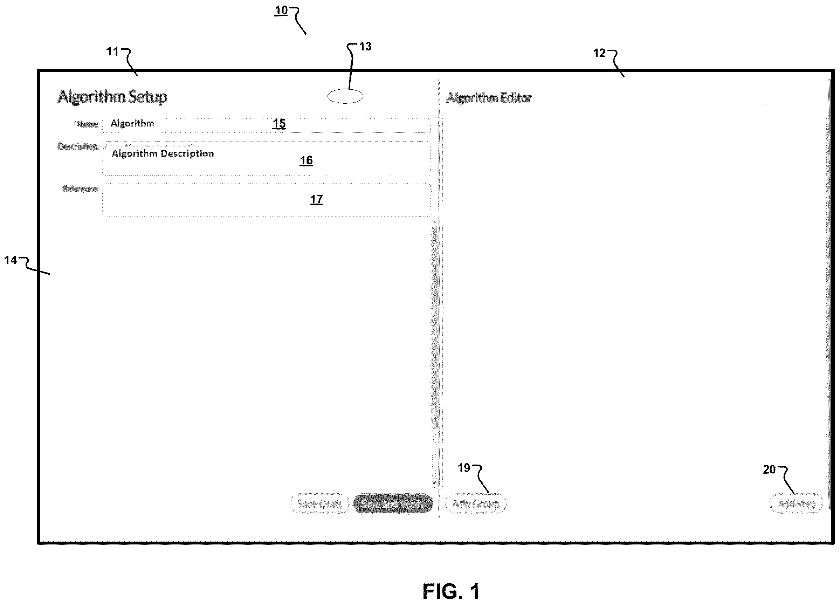

Figs. 1 to 9 show example configurations of a user interface (Ul) 10 that enables a user, such as a medical doctor or clinician, to build a medical algorithm of the type described herein. The UI may be a graphical user interface (such as a GUI), a text interface, or any other type of user interface. Referring to Fig. 1, example UI 10 includes a setup area 11 for a user to name and to characterize the medical algorithm, an editor box 12 for a user to input information that defines the medical algorithm, and a display area 14 for software executing the UI to display the algorithm that was configured based on the information input in the editor area 12. The layout of Ul 10 described herein is for illustration only and may be different than that shown.

In this example, setup area 11 contains a name field 15, a description field 16, and a reference field 17. Name field 15 receives an input from a user to name the medical algorithm. The name may be based on the clinical setting in which the medical algorithm is used, e.g., cardiovascular surgery, trauma, orthopedic surgery, liver surgery, transplantation, or obstetrics. Any textual moniker may be used. In this example, the generic name "Algorithm" is used. In some implementations, name field 15 may be configured to accept one or more images that identify the algorithm. Description field 16 receives, from a user, an explanation of the function of the medical algorithm. In this example, description field 16 is configured to accept text. In some implementations, description field 16 may be configured to accept images or other content used to describe the algorithm. An example of the other content may be a uniform resource locator (URL) that may link to a page describing the medical algorithm. Reference field 17 receives, from a user, information about reference materials relating to the algorithm, such as books, scholarly articles, medical journals, scientific publications, or the like providing background or other information relating to the algorithm. In addition to text, reference field 17 may configured to accept URLs, images, or other content. Information may be received into the fields from a user or from another computer program, for example.

In some implementations, name field 15, description field 16, and reference field 17 may have character or size limitations. For example, name field 15 may be limited to tens of characters, whereas description field 16 and reference field 17 may be limited to hundreds or thousands of characters. In some implementations, there are no limitations on the number of characters or the size of the content in these three fields.

In a particular non-limiting example, the medical algorithm may be a medical algorithm to diagnose or to treat liver disease. In this example, "Liver Algorithm" may be included in name field 15. Description field 16 may be a description of the type of liver disease that the "Liver Algorithm" is intended to diagnose or to treat, such as liver surgery, liver transplantation, or other bleeding situations in patients with chronic liver disease (e.g., gastrointestinal bleeding). Reference field 17 may include a list of books, articles, or journals containing information about the liver disease.

In some implementations, part of the algorithm setup may include the user entering patient information such as, but not limited to, a clinical setting such as type of surgery that the patient is undergoing or other medical treatment that the patient is undergoing, whether the patient is bleeding or has non-bleeding thrombosis, a stage of surgery, a medical condition of the patient, body weight of the patient, age of the patient, sex of the patient, a medical diagnosis for the patient, and/or a treatment that the patient has undergone or is undergoing. This patient information is associated with the medical algorithm built using Ul 10. The association can be made, for example, by a medical professional. For patients having those the characteristics or conditions contained in the patient information, the medical algorithm may be retrieved from a library of one or more of medical algorithms using a computer system.

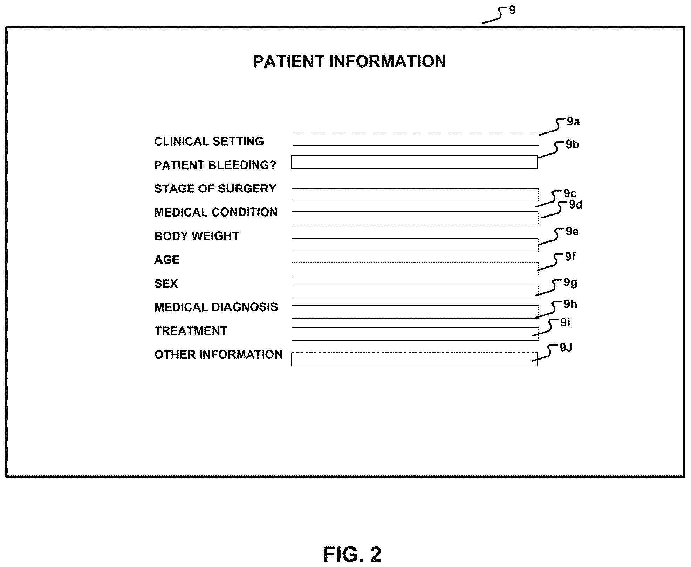

The patient information may be entered by a user or programmatically into a Ul. In some implementations, the patient information is extracted from an image (e.g., a scanned form or a screenshot) using optical character recognition software (OCR), and the extracted information is entered into the Ul. The UI may be accessed via a button, such as optional button 13 in setup area 11 or elsewhere, or through a pull-down menu or other control (not shown) associated with, but not contained on, UI 10. Fig. 2 shows an example of a UI 9 that may be used to enter patient information. In this example, UI 9 includes (i) a field 9a to indicate a clinical setting in which the medical algorithm is to be applied, such as cardiovascular surgery, trauma, orthopedic surgery, liver surgery, transplantation, or obstetrics, (ii) a field 9b to indicate whether a patient to which the medical algorithm is to be applied is bleeding or has non-bleeding thrombosis, (iii) a field 9c to indicate a stage of the surgery, (iv) a field 9d to indicate a medical condition of the patient, (v) a field 9e to indicate the body weight of the patient, (vi) a field 9f to indicate the age of the patient, (vii) a field 9g to indicate the sex of the patient, (viii) a field 9h to indicate a medical diagnosis for the patient, (ix) a field 9i to indicate a treatment that the patient has undergone or is undergoing (e.g., anticoagulation with heparin, low molecular weight heparin, vitamin K antagonists, direct thrombin inhibitors, direct factor Xa inhibitors, factor Xi inhibitors or treatment with antiplatelet drugs such as acetylsalicylic acid, P2Y12 inhibitors, PAR inhibitors, or GPllbllla receptor inhibitors), and (x) and a field 9j to indicate any other relevant information about the patient. Additional fields may be included in UI 9, or one or more fields may be omitted.

Referring back to Fig. 1, editor area 12 supports building a medical algorithm to be displayed in display area 14. Editor area 12 contains user-selectable prompts, such as buttons 19 (Add Group) and 20 (Add Step), which enable the user to create, to configure, and/or to revise a medical algorithm by adding groups, steps, and/or rules.

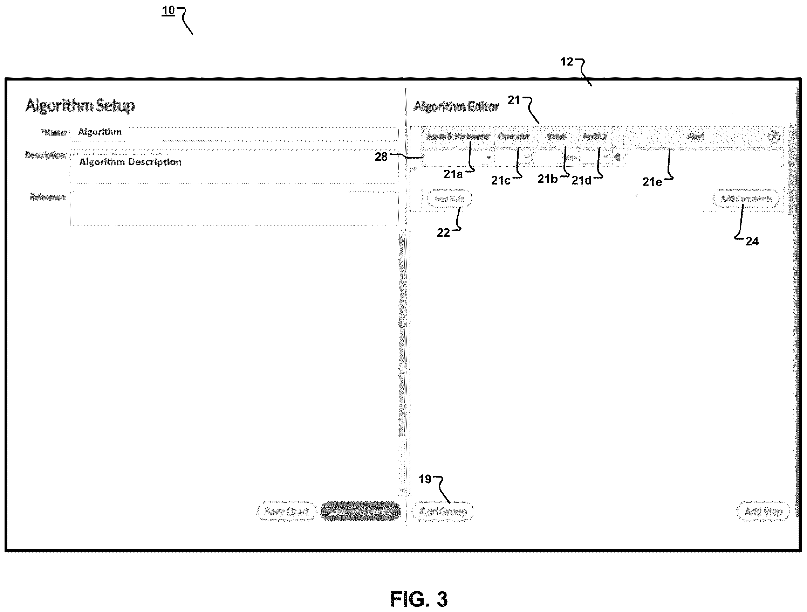

Button 19 (Add Group) is for adding a group to the medical algorithm. Referring to Fig. 3, user selection of button 19 causes Ul 10 to display a definition box 21 to define a step in a group, and to display associated prompts. The associated prompts include a button 22 (Add Rule) to add a rule to the step and a button 24 (Add Comments) to add comments to the step. The comments may explain the current rule and/or step and provide additional information about a treatment and/or diagnosis. The comments may be added in a pop-up text box (not shown) or in another region of UI 10 and accessed as described below. User selection of button 19 also indicates to UI 10 that all subsequently added content is for the current group until a new group is added.

An example definition box allows a user to define a step in the group and one or more rules for the step. As shown in Fig. 3, when initially displayed, a definition box 21 includes a single row 28 having empty fields for specifying a rule, and columns each for specifying a component of a rule in a corresponding row.

The columns include assay and parameter 21a column that allows the user to specify which parameter (P) values are to be evaluated by a rule and the assays (A) from which the parameter values have been obtained; column 21b allows the user to specify benchmarks / predefined values (e.g., AA, BB) against which the parameter values from column 21a are evaluated, operator 21c column allows the user to specify mathematical operators (e.g., "=", "<", ">", "≥", "≤") for the type of evaluation of the parameter values against the benchmarks / predefined values, conjunctions 21d column allows the user to specify conjunctions (e.g., "AND" or "OR") that indicate a relationship among the rules in a single step to produce an outcome, and notification 21e column allows the user to specify a notification such as a treatment and/or a diagnosis to be output in the event that the step is satisfied. In this example, content for assay and parameter column, the operator column, and the conjunctions column may be selected by the user using drop-down menus, whereas content for the value column and the notification column may be typed into their respective fields by the user. In some implementations, content for the assay and parameter column, the operator column, and the conjunctions column may be typed into their respective fields by the user.

The notification, or outcome, can include a summary, a diagnosis and/or a treatment option. The summary can include any comment that provides information related to the evaluation of the step or steps in a group. For example, the summary can describe what an analysis is showing, for example, "prolonged clotting time". The diagnosis could be hyperfibrinolysis and the treatment option can be tranexamic acid. One or more of the diagnoses and treatment options can be included in the notification. Specific examples are provided below.

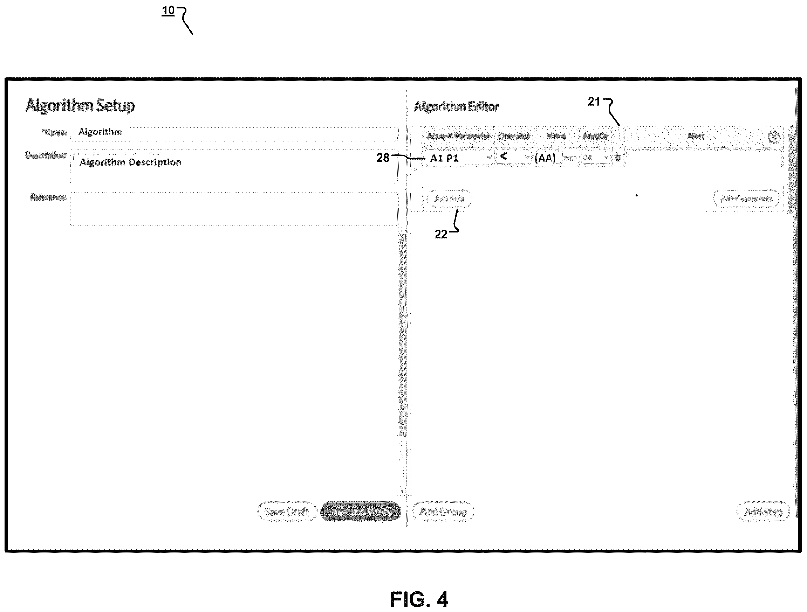

Referring to Fig. 4, a user has input content to definition box 21 to define a rule in row 28. The content includes a parameter P1 from assay A1 (A1 P1) to be evaluated by the rule, a predefined value (AA) with units (millimeters (mm) in this example) against which A1P1 is to be evaluated, and a mathematical operator "<" that defines the type of evaluation. The content also includes an "OR" in the conjunction field, which means that the step defined by definition box 21 will include more than one rule. In this example, no entry in the conjunction field means that no additional steps are to be added.

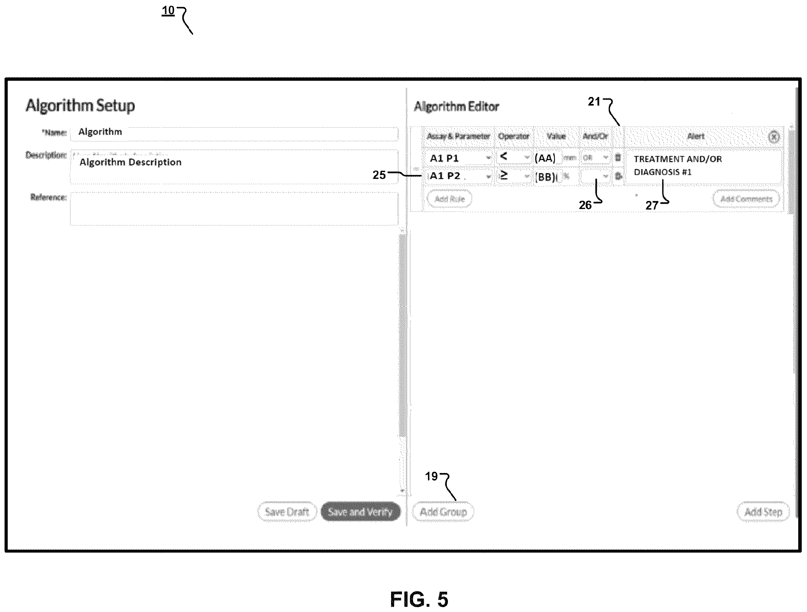

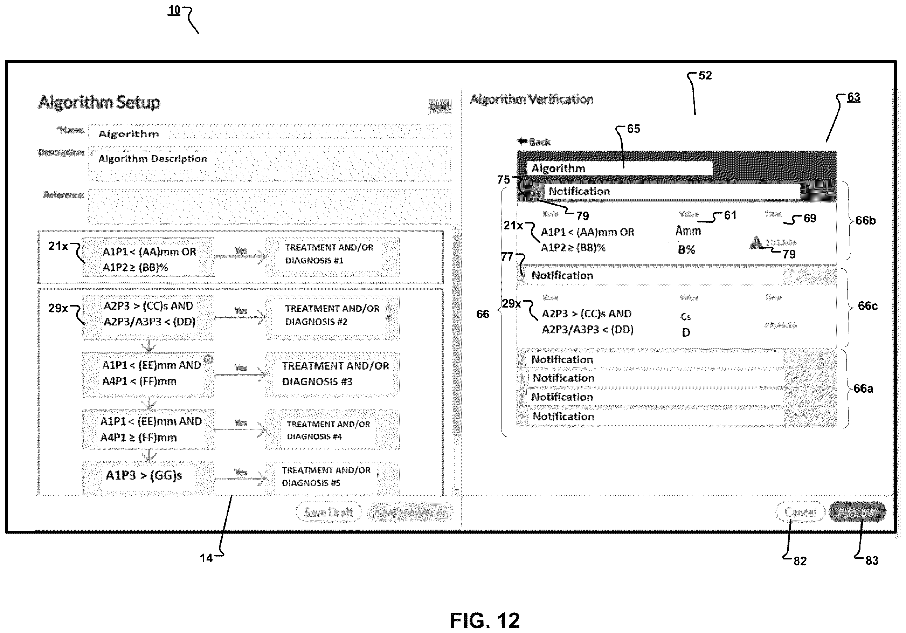

To add the next rule to definition box 21, the user selects button 22 (Add Rule). Referring also to Fig. 5, this selection causes UI 10 to display row 25 in definition box 21. Row 25 is initially blank like row 28 in Fig. 3. Fig. 4 shows row 25 after content for the next rule has been added. No conjunction is included in conjunction field 26. This means that no additional rules are to be added to the step defined by definition box 21. The content added to notifications field 27 identifies a notification that is recommended when a step defined by definition box 21 is not satisfied. The notification includes a diagnosis and treatment. In example UI 10, the notification is always displayed regardless of whether or not the step is satisfied to allow a clinician to know the goal of the step. For example, if the step is not satisfied, the notification may be displayed in grey. If the step is satisfied, an alert is triggered and displayed with the notification. Alerts are described in more detail below with reference to FIG.12.

In this example, the step defined by definition box 21 includes first rule A1 P1 <(AA)mm, where P1 is the parameter, A1 is the assay that determined the parameter, and (AA) is the predefined value in millimeters (mm) against which A1P1 is evaluated, here whether A1P1 is less than AA. In this example, the step defined by definition box 21 includes second rule A1P2≥(BB)%, where P2 is the parameter, A1 is the assay that determined the parameter, and (BB) is the predefined value in percent against which A1P2 is evaluated, here whether A1P2 is greater than or equal to BB. The two rules are related by the "OR" conjunction, meaning that if either rule is satisfied, the medical algorithm will display an alert along with the treatment and/or diagnosis #1 from notification 27 field. If neither rule is satisfied, the notification may be displayed but not triggered with an alert.

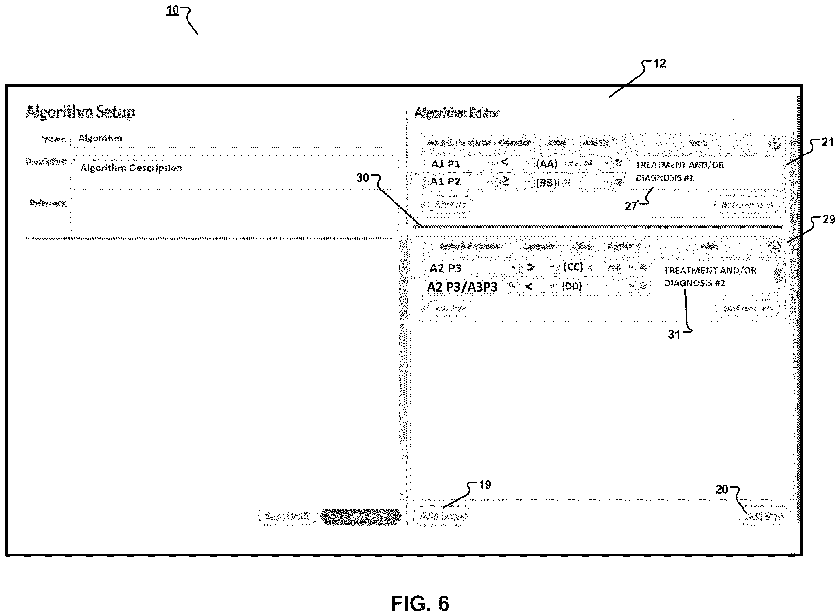

Referring also to Fig. 6, user selection of button 19 (Add Group) causes Ul 10 to display a new definition box 29 into which the user may input content for a step in a second group, which is different from the step/group defined by definition box 21. Definition box 29 may be configured and populated in the same manner as definition box 21. The second group defined, at least in part, by definition box 29 is separated from the step/group defined by definition box 21 in editor area 12. In this example, that separation is implemented by a line 30; however, a spacing or other markings may indicate the separation.

The relationships among the parameters, operators, predefined values, conjunctions, and notifications in definitions box 29 follows the same pattern as those for definitions box 21. In this example, the step defined by definition box 29 includes rule A3P3>(CC)s, where P3 is the parameter, A3 is the assay that determined the parameter, and (CC) is the predefined value in second(s) against which A3P3 is evaluated, here whether A3P3 is greater than (CC). In this example, the step defined by definition box 29 includes rule A2P3/A3P3<(DD), where A2P3/A3P3 is a combination of parameters, A2 and A3 are the different assays that determined different values of parameter P, and (DD) is the predefined value against which A2P3/A3P3 is evaluated, here whether A2P3/A3P3 is less than (DD). The two rules are related by the AND conjunction, meaning that both rules must be satisfied for the medical algorithm to display an alert along with the treatment and/or diagnosis #2 from notification 31 field. If either rule is not satisfied, the notification may be displayed but not triggered with an alert.

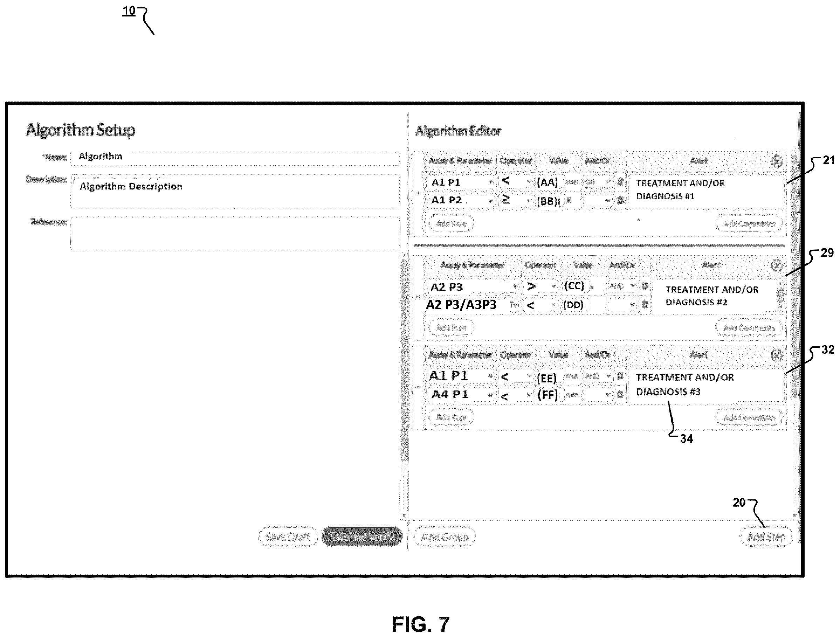

Referring also to Fig. 7, user selection of button 20 (Add Step) causes UI 10 to display definition box 32 to define a second step in the second group. Definition box 32 may be configured and populated in the same manner as definition boxes 21 and 29.

The relationships among the parameters, operators, predefined values, conjunctions and notifications in definitions box 32 follows the same pattern as those for definitions boxes 21 and 29. In this example, the step defined by definition box 32 includes rule A1P1<(EE)mm, where P1 is the parameter, A1 is the assay that determined the parameter, and (EE) is the predefined value in millimeters against which A4P1 is evaluated, here whether A4P1 is less than (EE). In this example, the step defined by definition box 32 includes rule A4P1<(FF)mm, where P1 is the parameter, A4 is the assay that determined the parameter, and (FF) is the predefined value against which A4P1 is evaluated, here whether A4P1 is less than (FF). The two rules are related by the AND conjunction, meaning that both rules must be satisfied for the medical algorithm to display an alert along the treatment and/or diagnosis #3 from notification 34 field. If either rule is not satisfied, the notification may be displayed but not triggered with an alert.

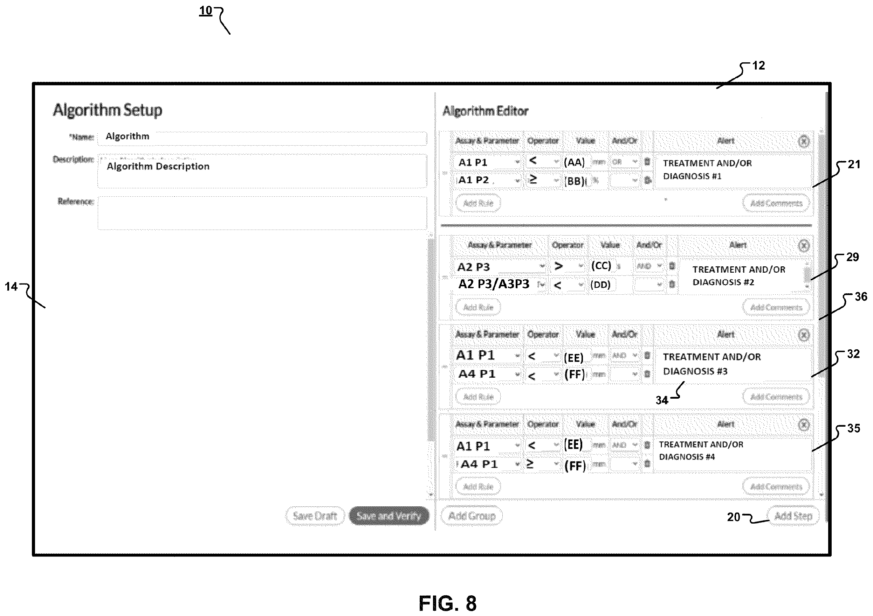

Referring also to Fig. 8, user selection of button 20 (Add Step) causes UI 10 to display definition box 35 to define a third step in the second group. Definition box 35 may be configured and populated in the same manner as definition boxes 21, 29, and 32.

The relationships among the parameters, operators, predefined values, conjunctions and notification in definitions box 35 follows the same pattern as those for definitions boxes 21, 29, and 32. In this example, the third step defined by definition box 35 includes rule A1P1<(EE)mm, where P1 is the parameter, A1 is the assay that determined the parameter, and (EE) is the predefined value in millimeters against which A1P1 is evaluated, here whether A1P1 is less than (EE). In this example, the third step defined by definition box 35 also includes rule A4P1≥(FF)mm, where P1 is the parameter, A4 is the assay that determined the parameter, and (FF) is the predefined value against which A4P1 is evaluated, here whether A4P1 is greater than or equal to (FF). The two rules are related by the AND conjunction, meaning that both rules must be satisfied for the medical algorithm to display an alert and the treatment and/or diagnosis #3 from notification 34 field. If either rule is not satisfied, the notification may be displayed but not triggered with an alert.

Additional steps and/or groups may be added in the editor area above. UI 10 may generate and display scroll bar 36 to allow a user to navigate to different definition boxes. Furthermore, although the example steps shown in Fig. 8 each contain two rules, a step may contain any number of rules, such as one rule, three rules, four rules, five rules, and so forth. Still further, a group may contain any number of steps such as one step (shown), two steps, three steps, four steps, five steps, and so forth.

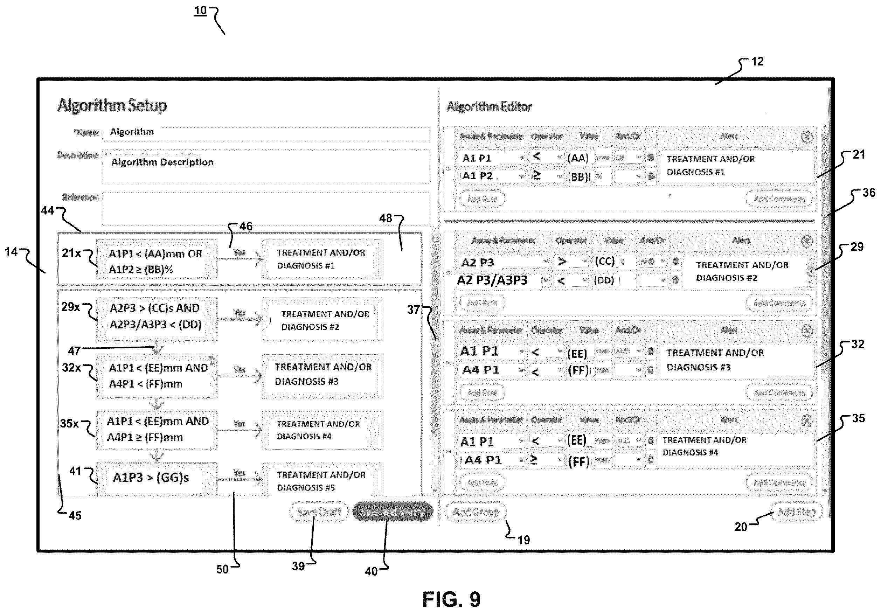

Referring to Fig. 9, display area 14 depicts, graphically, components of the medical algorithm created in editor area 12. In particular, Ul 10 generates the content for display in display area based on user input to the definitions boxes, including definitions boxes 21, 29, 32, and 35. Display area 14 also contains scroll bar 37 and prompts including button 39 (Save Draft) and button 40 (Save and Verify).

In some implementations, UI 10 automatically updates display area whenever any content defining the medical algorithm is provided in a definitions box. Thus, in such examples, display area is populated as soon as content is added to editor area 12. In some implementations, UI 10 may display graphical content in display area 14 as each new group is added. In some implementations, UI 10 may display graphical content in display area 14 as each new step is added. In some implementations, UI 10 may display graphical content in display area 14 as each new rule is added. Button 39 is for saving a draft of the medical algorithm to memory. For example, user selection of button 39 also causes UI 10 to save a current version of the medical algorithm in memory, from which it may be retrieved and revised later.

Button 40 is for saving the current version of the medical algorithm in memory as a version and for verifying that version. Verification is described below.

In this example implementation, display area 14 displays the content of step 21x which is defined by definitions box 21, the content of step 29x which is defined by definitions box 29, the content of step 32x which is defined by definitions box 32, and the content of step 35x which is defined by definitions box 35. Display area 14 also displays step 41, which is defined by a definition box that is not visible in the editor area 12, but which may be viewed using scroll bar 36.

Display area 14 identifies individual groups and also which steps are in which groups. In this example, this is implemented by frames such as frames 44 and 45, around steps in different groups. However, steps in different groups may be identified by lines, spacing, markings, text, or other indicia.

Display area 14 identified a notification that is to be displayed in response to executing a step by arrow and YES 46, as shown with respect to step 21x. Display area 14 indicates the sequence in which steps in a group are executed using arrows, such as arrow 47.

In an example implementation, the parameters, assays, predefined values, and notifications shown in the portion of the medical algorithm displayed in display area 14 of Fig. 9 may include the following.

For step 21x/group 48: P1 may be the "A5" clot firmness parameter obtained from the ROTEM^{®} *sigma* system or the ROTEM^{®} *delta* system by Werfen^{®}; A1 may be the EXTEM assay performed on the ROTEM^{®} *sigma* or the ROTEM^{®} *delta* systems by Werfen^{®}, where EXTEM tests the extrinsic pathway, uses tissue factor as an activator, and provides information about the time it takes for a test sample to clot; (AA) may be 35mm; P2 may be the "ML" parameters obtained from the ROTEM^{®} *sigma* system or the ROTEM^{®} *delta* system by Werfen^{®}; (BB) may be ≥15%; and treatment and/or diagnosis #1 may include a pathology of "clot instability", a diagnosis of hyperfibrinolysis, and/or a treatment of "tranexamic acid with a dose of 25 mg/kg as a single bolus", meaning 25 milligrams (mg) per kilogram (kg) of a patient's body weight is to be administered in a single dose.

For step 29x/group 50: P3 may be the "CT" parameter obtained from the ROTEM^{®} *sigma* system or the ROTEM^{®} *delta* system by Werfen^{®}; A2 may be the INTEM assay performed on the ROTEM^{®} *sigma* system or the ROTEM^{®} *delta* system by Werfen^{®}, where INTEM tests the intrinsic pathway, uses phospholipid and ellagic acid as activators, and measures how long it takes blood to form a clot; (CC) may be 240s; A3 may be the HEPTEM assay performed on the ROTEM^{®} *sigma* or the ROTEM^{®} *delta* systems by Werfen^{®}, where HEPTEM is similar to INTEM but uses lyophilized heparinase to neutralize heparin, and reports a result that uncovers any coagulopathy that may coexist alongside heparinization; (DD) may be ≥1.25; and treatment and/or diagnosis #2 may include a pathology of "increased INTEM/HEPTEM CT-ratio due to heparin", a diagnosis of heparin effect, and/or a treatment of "protamine with a dose of 15-50 mg (2.5-5ml) per 80 kg body weight and reassess ACT and INTEM/HEPTEM after the therapeutic intervention".

For step 32x/group 50: P1 may be as defined above; A1 may be as defined above; EE may be 30mm; A4 may be the FIBTEM assay performed on the ROTEM^{®} *sigma* system or the ROTEM^{®} *delta* system by Werfen^{®}, where the FIBTEM assay is used to monitor the blood coagulation process of citrated whole blood samples after activating the extrinsic pathway, blocking platelet contribution to clot firmness, and eliminating a heparin-like effect; FF may be ≤9mm; and treatment and/or diagnosis #3 may include a pathology of low fibrin contribution to clot firmness, a diagnosis of hypofibrinogenemia, and/or a treatment of "Cryoprecipitate or Fibrinogen Concentrate administration with a target of A5 FIBTEM ≥ 12 mm.

For step 35x/group 50: P1 may be as defined above; A1 may be as defined above; EE may be as defined above; A4 may be as defined above; FF may be as defined above; and treatment and/or diagnosis #4 may include a pathology of "low platelet contribution due to clot firmness, a diagnosis of "thrombocytopenia" and/or a treatment of "Platelet concentrate transfusion with a dose of 1-2 pooled or apheresis platelet concentrates in an adult patient".

For step 41/group 50: P3 may be as defined above; A1 may be as defined above; (GG) may be 80s; and treatment and/or diagnosis 5 may include a pathology of "prolonged extrinsic clotting time, a diagnosis of reduced thrombin generation by the extrinsic pathway, and/or a treatment of "PCC 15-25 IU/kg bw or FFP 10-15 mL/kg bw", where PCC (prothrombin complex concentrate) and FPP (fresh frozen plasma) are treatments.

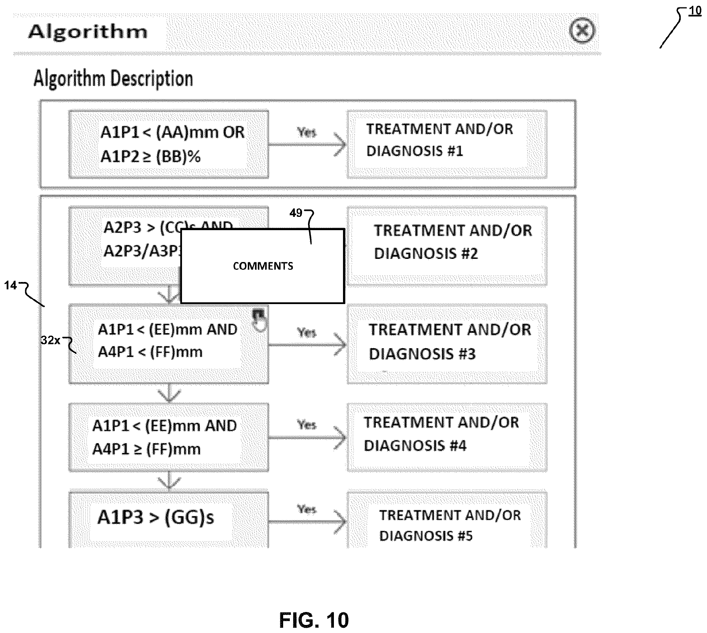

Fig. 10 shows how that Ul 10 automatically generates a pop-up window showing comments added by a user during algorithm building, as described above. More specifically, a user may select (e.g., click-on) a representation of a step and, in response, UI 10 displays a pop-up window-49 containing the comments for that step. Selecting a blank region of Ul 10 may cause Ul 10 to remove pop-up window 49.

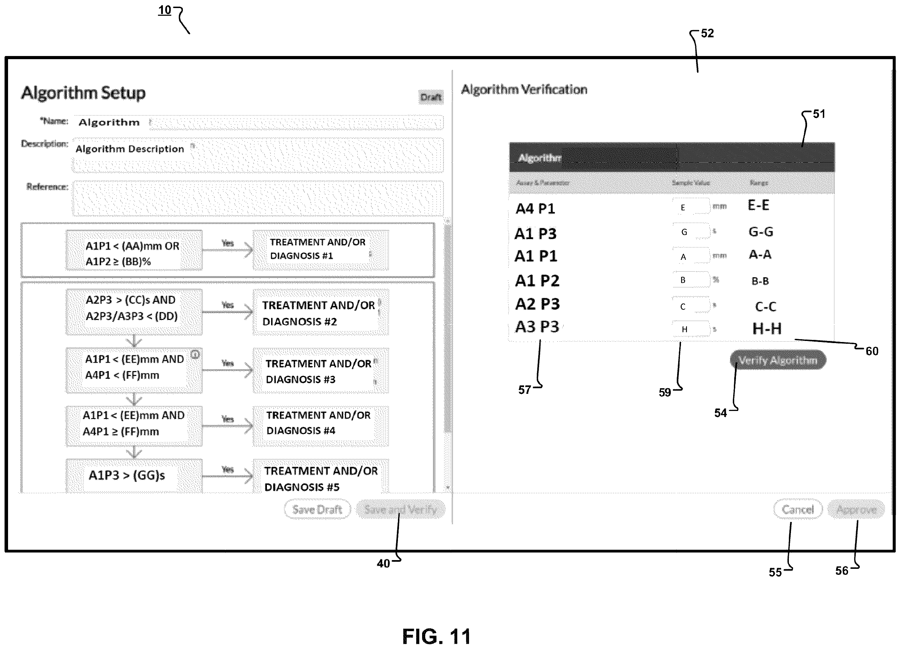

Referring to Fig. 11, user selection of button 40 causes UI 10 to replace editor area 12 with verification area 52 and to display an algorithm verification box 51 in verification area 52. Algorithm verification box 51 is for receiving parameter values against which the medical algorithm may be evaluated. Algorithm verification area 52 also contains button 54 (Verify Algorithm) to initiate algorithm verification, button 55 (Cancel) to cancel the verification prior to initiation, and button 56 (Approve) to approve of the medical algorithm following verification. Buttons 54, 55, and 56 may be selected by a user to implement the corresponding action.

To generate algorithm verification box 51, software that generates Ul 10 extracts parameters from the medical algorithm defined by the definition boxes in editor area 12. For example, those parameters may correspond to the parameters from the assay and parameter fields of the definitions boxes, and may be retrieved from memory. The parameters are listed in a column 57 along with corresponding fields 59 for entering a value of each of those parameters, with units for that parameter displayed alongside each field. The values may be entered by a user or by a computer program, including an optical character recognition program that extracts the values from an image. Also displayed are a list of acceptable ranges 60 for a corresponding parameter. The acceptable ranges may be defined by a medical professional and may specify ranges of the parameter that are considered normal or that do not require intervention. Values outside of those ranges cannot be entered into the corresponding field in some implementations. If a value outside of an acceptable range is entered into a corresponding field, the UI may display an error message (not shown) when verification is initiated via button 54. The user may then enter a value within the acceptable range to proceed with verification.

The values entered into fields 59 may be mock test result values, meaning that the values are provided for verification purposes, and may not be actual test result values generated by a test system. In some implementations, the values may be from prior tests that are used for verification purposes only. In an example, the parameters (P) and assays (A) are as defined above. Examples of mock test result values and corresponding ranges for this example may be as follows: for A4P1 the mock test result value may be 5 and the range may be 2-33; for A1P3 the mock test result value may be 50 and the range may be 45 -172; for A1P1 the mock test result value may be 44 and the range may be 13-87; for A1P2 the mock test result value may be 55 and the range may be 0-100; for A2P3 the mock test result value may be 200 and the range may be 123-365; and for A3P2 the mock test result value may be 200 and the range may be 122-376. Other mock test result values and ranges may be used instead of these.

Upon user selection of button 54 to initiate verification, the medical algorithm is executed using the mock test values and the results of that execution are displayed by UI 10. For example, referring to Fig. 12, Ul 10 displays a report 63 in algorithm verification area 52, along with button 82 (Cancel) to cancel the verification, and button 82 (Approve) to approve of the medical algorithm following verification.

Report 63 is displayed adjacent to the representation of the medical algorithm in display area 14. Report 63 shows the result of execution of the medical algorithm using the mock test result values input into algorithm verification box 51. In this example, report 63 identifies the name 65 of the medical algorithm; displays steps such as 21x, 29x of the medical algorithm graphically in expandable and collapsible boxes, such as boxes 66, along with the treatment and/or diagnosis for the corresponding step and, if applicable, an alert 79 if a condition of the corresponding step has been satisfied (indicating that the treatment and/or diagnosis associated with the corresponding step should be applied), the time 69 that a corresponding step was completed, and values 62 that were analyzed the corresponding steps. The boxes may be expanded or collapsed by clicking anywhere in a box. Drop-down arrows, such as arrow 77, are used as visual indicators to indicate whether a box has been expanded or collapsed.

In the example of Fig. 12, when expanded by user selection, box 66b displays step 21x graphically, which includes the rules contained in the step and the mock test values against which the corresponding rules are evaluated (e.g., Amm or B%). In the example of Fig. 12, when expanded, box 66c displays step 29x graphically, which includes the rules contained in the step and the mock test values against which the corresponding rules are evaluated. Boxes 66a are collapsed in the example of Fig. 12 and, therefore, boxes 66a do not show their content; however, those boxes may be expanded. In this example, the mock test result values evaluated by step 21x triggered the alert 79, which means that the mock test result values, when evaluated, satisfied the conditions of the step 21x. In this example, this means that Amm<(AA)mm OR B%≥(BB)%. In this example, alert 79 is represented by a triangle containing an exclamation point; however, any graphic may be used. In this example, box 66b is also colored a distinguishing color, such as red, or shaded differently from boxes for steps that do not generate alerts, to indicate that there was an alert produced by the step in box 66b. By contrast, the mock test result values evaluated by step 29x did not trigger an alert, which means that the mock test result values for step 29x did not satisfy the conditions of the step. In this example, this means that Cs≤(CC) AND D≥(DD). Since no alerts was generated by step 29x, box 66c does not display an alert and box 66c may be colored or shaded differently from boxes for steps that do generate alerts.

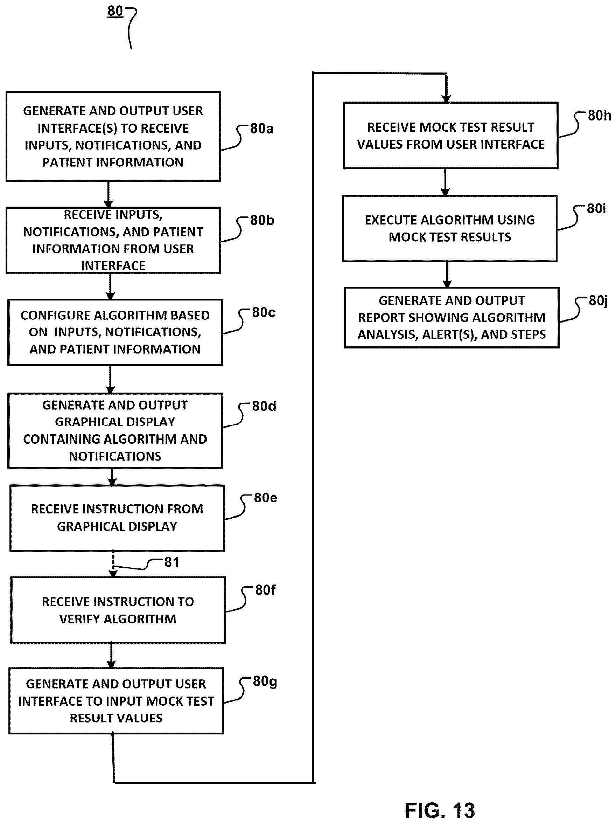

Fig. 13 is a flowchart showing example operations included in an example process 80 for building and verifying a medical algorithm by generating, displaying, interacting with, and otherwise using example Uls such as those described with respect to Figs. 1 to 12. Process 80 may be performed by a control system, such as a computing system, examples of which are described herein.

Process 80 includes generating data for, and outputting, (80a) Uls configured to receive inputs such as parameters, predefined values, operators, notifications, clinical information, and patient information that are used to build the medical algorithm. Examples of the Uls output by process 80 includes UI 9 to receive patient information and UI 10 containing setup area 11, editor area 12, and display area 14. The configuration of the Uls, such as the number of definitions boxes may be based on user input. For example, as the user adds more groups and/or steps to the medical algorithm, additional definition boxes may be added in which the user may input information from which to build the medical algorithm.

Process 80 receives (80b), from the Uls output in operation 80a, inputs including the patient information and clinical information described herein. The inputs may include, as described above, parameter values to be evaluated by a rule and the assays from which the parameter values have been obtained, benchmarks / predefined values against which the parameter values are evaluated, mathematical operators for a type of evaluation of the parameter values against the benchmarks / predefined values, conjunctions indicating a relationship among rules in a single step to produce a notification, and notifications, such as a treatment and/or a diagnosis to be output by the medical algorithm and highlighted if the medical algorithm generates an alert.

Process 80 configures (80c) the medical algorithm based on the inputs, notifications, and patient information received in operation 80b. For example, process 80 arranges the parameters values, the mathematical operators, the benchmarks / predefined values, the conjunctions, and the notifications to produce the rules, steps, and/or groups described herein, and generates executable instructions to implement those, steps, and/or groups as part of a computer program. For example, referring to notification box 21 (see, e.g., Fig. 9), process 80 arranges A1P1, ≤, (AA)mm, OR, A1P2, ≥, (BB)%, and treatment and/or diagnosis #1 to produce step 21x and generates executable instructions to evaluate that step. Configuring the medical algorithm also includes associating the patient information and clinical, such as that provided in UI 9 (Fig. 2), with the medical algorithm. For example, process 80 may store all or some of the patient information with a pointer to the medical algorithm so that, in response to input of all or some of this information, the medical algorithm associated with the patient information can be retrieved and executed as described below. In some implementations, all or some of the patient information may be stored in a look-up table that associates the patient information to one or more medial algorithms.

Process 80 generates data for, and outputs, (80d) a graphical display or textual representation of the medical algorithm and corresponding notifications, such as the medical algorithm and corresponding notifications shown in display area 14 of Fig. 9. The graphical display or textual representation of the medical algorithm may update as new information is entered into the definitions boxes as described herein. For example, as each rule, step, or group is configured (80c) by process 80, process 80 may generate a corresponding graphical or textual display of that step in display area 14. As additional steps are configured, process 80 may generate a corresponding graphical or textual display of those steps step in display area 14. In other words, in these examples, process 80 generates graphical or textual displays of parts of the medical algorithm as those parts are completed in the algorithm editor, and need not wait for a user input to generate the graphical or textual display of the medical algorithm.

Process 80 receives (80e), in response to user input on UI 10, an instruction corresponding to an action to take with respect to the medical algorithm defined by UI 10. Examples of actions that may be taken include, but are not limited to, verifying the medical algorithm, storing the medical algorithm in memory, or editing the medical algorithm.

In an example, the medical algorithm may be verified (80f). Operations to verify the medical algorithm are optional in process 80, as indicated by the dashed arrow 81 preceding operation 80f. To verify the medical algorithm in this example, a user selects button 40 (Fig. 9). In response to the selection, process 80 receives (80f) the instruction to save and verify the medical algorithm. Process saves the current version of the medical algorithm in memory if it is not already saved. Process 80 generates data for, and outputs, (80g) a UI configured to receive mock test results values that the saved medical algorithm processes. For example, process 80 generates data for, and outputs, algorithm verification box 51 of Fig. 11. The user or a computer program provides mock or prior test result values into all fields 59 of algorithm verification box 51 or into a subset of those fields of algorithm verification box 51 corresponding to groups to be verified (for example, if only a subset of groups is to be verified).

Following input of the mock or prior test results values, the user selects verify algorithm button 54 of Fig. 11. In response, process 80 receives (80h) the input mock test result values from Ul 10 and executes (80i) all or part of the medical algorithm using the input mock test result values. Process 80 generates data for, and outputs, (80j) a report showing steps in the medical algorithm and a result of execution of the medical algorithm using the mock test result values. Fig. 12 shows an example of such a report 63, which is displayed in algorithm verification box 52 next to display area 14 that displays the medical algorithm. The contents of example report are described above.

Actual verification may be performed manually by the user based on the report. For example, the user determines whether the notifications and alerts, in the report make sense for the medical algorithm that the user intended to build. If the user determines that the notifications and alerts, in the report make sense, the user may select button 83 (Approve), in response to which process 80 stores an indication that the medical algorithm has been approved following verification. The indication may be stored in association with the medical algorithm or in a table or the like listing medical algorithms built according to process 80. If the user determines that the notifications and alerts, in the report do not make sense, the user may select button 82 to cancel the current verification, which may result in re-display editor area 12 (Fig. 9) and the content thereof.

Process 80 may be used to build, and to store in computer memory, multiple medical algorithms. The multiple medical algorithms may constitute a library of medical algorithms. In some implementations, different medical algorithms in the library may be associated with different patient or clinical information. For example, some medical algorithms may be usable for men, some medical algorithms may be used for women; some medical algorithms may be for a particular type of surgery that a patient is having such as liver surgery; some medical algorithms may be used if a patient is bleeding, and so forth. Medical algorithms from the library may be retrieved for execution using patient information and/or clinical information as described herein. In some implementations, the notifications in an algorithm can include a recommendation to use one or more additional algorithms.

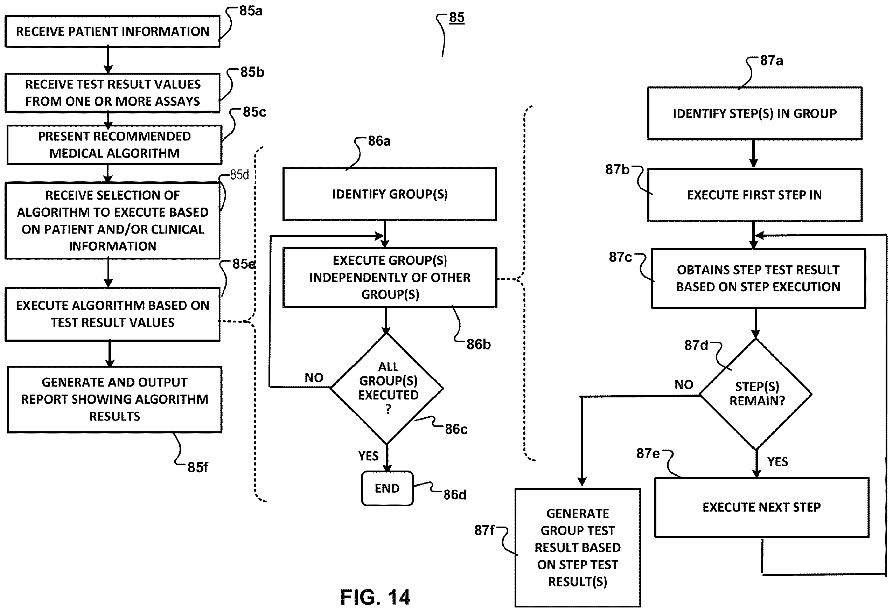

Fig. 14 shows example operations included in an example process 85 for executing a medical algorithm built according to process 80. Process 85 may be performed by a control system, such as a computing system, examples of which are described herein.

Process 85 receives (85a) patient information such as that described with respect to UI 9. For example, the patient information may be input into a user interface by a user or otherwise provided by a user or a computer program. In some implementations, the patient information is extracted from an image such as a scanned form or a screen shot using an optical character recognition algorithm.

Process 85 receives (85b) test result values, such as those described above. The test result values may be received from memory or a test system, examples of which include, but are not limited to, those described herein. The test result values may be result(s) of one or more assays performed on the test system. In some implementations, process 85 receives, from the test system, one or more images which may containing metadata. The image(s) may show reaction curves for the one or more assays, which show elasticity over time when a blood clot forms or dissolves. The metadata in an image includes the test result values for the assay that produced the reaction curve in the image. Process 85 extracts the metadata from these image(s). In some implementations, the test result values may be provided to process 85 manually - for example, by a user - or the test result values may be provided to process 85 through electronic communication between the control system executing process 85 and the test system. In some implementations, the images include one or more test result values which are not included in the metadata. In these instances, the test data can be extracted using optical character recognition algorithms. In some implementations, a graph or other image may be analyzed by an algorithm to extract one or more test result values.

Some test result values may be provided in their final form for evaluation by the medical algorithm. For example, test result values for the ROTEM^{®} A10, and A20 parameters (and potentially others) are provided in their final form since intermediary values of such parameters may not be helpful to determine treatment and/or diagnosis using the medical algorithm. Some test result values may be provided in their non-final form for evaluation by a medical algorithm. For example, test result values for the ROTEM^{®} ML parameter (and potentially others) may be provided in their non-final form, since intermediary values of such parameters may be helpful for treatment and/or diagnosis using the medical algorithm.

Process 85 presents a recommended medical algorithm based on the patient and/or clinical information. For example, if the patient and/or clinical information indicates that the patient is a woman undergoing a liver transplant and is bleeding, process 85 may present (85b) a recommended medical algorithm that is associated with such information to a user for selection along with a description thereof, such as the clinical setting in which the medical algorithm may be used and the types of patients for which the medical algorithm may be used. The user may the elect to select the medical algorithm for execution.

Process 85 receives (85c) a selection of medical algorithm to execute based on the received (85a) patient and/or clinical information. After receiving selection (85c) of the medical algorithm, process 85 executes (85e) the medical algorithm using the received test result values. When executing (85d) the medical algorithm, process 85 identifies (86a) groups in the medical algorithm. The groups may be identified based on information identifying each group added to the medical algorithm in response to selecting button 19 (Add Group) when building the medical algorithm. For example, frames 44 or 45 (Fig. 9) or other information separating the medical algorithm may be stored in association with the medical algorithm and used to identify the groups.

Process 85 executes (86b) each group in the medical algorithm independently of other groups in the medical algorithm. In other words, process 85 executes (86b) the steps and rules in a given group independently of the steps and rules in other groups. Independent execution may include parallel execution, where at least part of the steps and rules in a given group are executed concurrently with execution of at least part of the steps and rules in in one or more other groups of the medical algorithm. For example, referring to Fig. 9, at least part of the steps and rules in group 50 may be executed at a same time as the step and rule of group 48. In some implementations, independent execution may include steps and rules in one group executing after all steps and rules in another group have been executed. In the example of Fig. 9, steps 21x and 29x may execute concurrently, but steps 32x and 35x of group 50 may execute after steps 21x and 29x. In some implementations, steps and rules from two, three, four, five, six, or more groups may be executed independently.

To execute (86b) each group, process 85 identifies (87a) steps in the group. The steps may be identified based on information identifying each step that may be incorporated into the medical algorithm in response to selecting button 20 (Add Step) (e.g., Fig. 9) when building the medical algorithm.

Process 85 executes steps in each group in a sequence in which the steps are arranged in the group. For example, if a group contains only one step, that one step is executed. If a group contains a series of steps, process 85 executes the steps in the series in the sequence in which the steps are arranged in the series. For example, in group 50 (Fig. 9), step 29x is executed, followed by step 32x, and so forth. Process 85 executes (87b) the first step in a group. Executing a step, including the first step, includes evaluating one or more test result values against one or more rules contained in each step. In cases where a final test value for a parameter is required to execute a step, and that final test value has not yet been received, the medical algorithm will wait for that final test value to execute the step before proceeding to a next step.

In some implementations, when a rule in a step indicates that a value is less than or equal to a predefined value, then the rule continues to be evaluated until a test result value is finalized. For example, if the conditions of a step are not satisfied, then a rule in the step can be evaluated repeatedly while the value is updated. For example, ML keeps growing for the duration of the algorithm so if a rule is testing whether > 15 %, then just because the ML is currently 10% does not mean that the rule has completed its evaluation. In this example, the medical algorithm continues to evaluate the step until the user ends the algorithm or ML exceeds 15 %.

In some implementations, after a step has been executed, that step will not be executed again unless (i) new data is received or (ii) the step is executing using test result values that need not be finalized, such as ROTEM^{®} ML test result values.

Process 85 obtains (87c) the step test result based on execution of the step. In this example, the obtained step test result includes a treatment and/or diagnosis, such as treatments and/or diagnosis #1 to #5 of Fig. 9 and whether an alert should be triggered.

Process 85 determines (87d) whether one or more additional step(s) remain in a group. This may be determined based on the information identifying each step incorporated into the medical algorithm in response to selecting button 20 (Add Step) when building the medical algorithm, as described above. If an additional step remains in the group, process 85 executes (87e) the next step in the group in sequence. For example, referring to Fig. 9, in group 50, step 29x is evaluated first, followed by step 32x, followed by step 35x, and so forth. For each step, if a parameter is needed for evaluation, process 85 will wait for that parameter and will not proceed to the next step. Operations 87c to 87e continue for a group until all steps in the group have been executed.

Process 85 generates data for, and outputs (87f), a group test result, which includes the step test results for each step in a current group. The groups test results are incorporated into a report output by process 85, as described below.

Process 85 determines (86c) if all groups have been executed. This determination (86c) includes whether all steps and rules in each group have continued executing. If all groups have not been executed, process 85 continues executing the groups; otherwise, group execution ends (86d).

Process 85 generates data for, and outputs, (85f) a report showing results of the medical algorithm. The report may have a format that is similar to the format of report 63 shown in algorithm verification area 52 of Fig. 12.

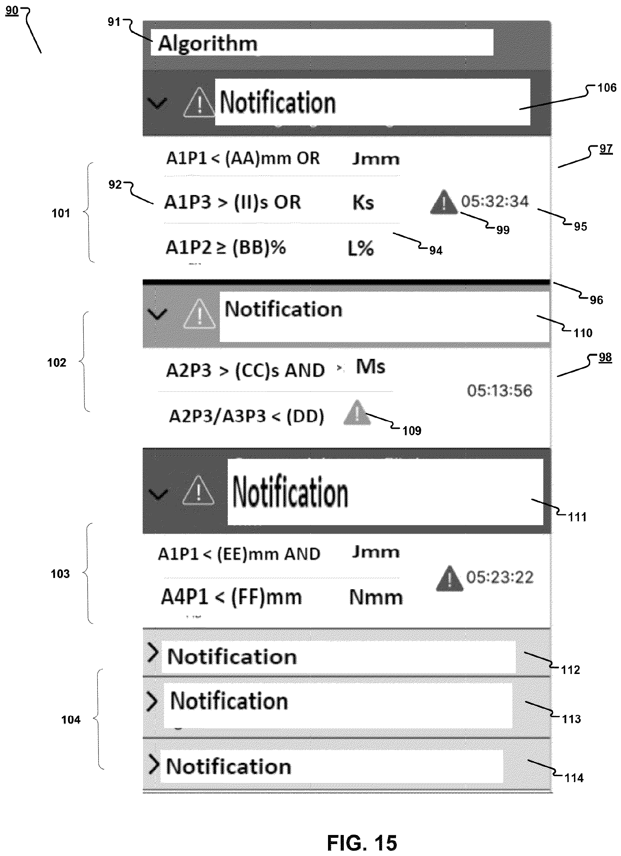

Fig. 15 shows an example of a report 90 generated and output (85f) by process 85. In this example, report 90 includes the name 91 of the medical algorithm, the content 92 of each step (e.g., rules), the test result values 94 for each step, a time 95 that a step was evaluated, a line 96 separating one group 97 from another other group 98 in the medical algorithm, and a box 106 for each step indicating a notification (e.g., a treatment or diagnosis) for each step. In some implementations, the notification can be a recommendation to use one or more other algorithms. Alerts are indicated by triangles, such as triangle 99 and specific colors or shading (e.g., red) for a step that generated the alert.

In example report 90, steps 104 did not generate alerts and, therefore, are colored or shaded differently than steps 101 and 103. Steps 101 and 103 generated alerts. The alerts generated by steps 101 and 103 are a result of test result values satisfying the condition or conditions required by the corresponding step. Step 102 also generated an alert, which is indicated by triangle 109. However, the alert generated by step 102 is different from that generated by steps 101 and 103. More specifically, in some jurisdictions, government agencies limit the test result values that can be reported by the system. In this example, report 90 indicates that test result value for step 102 is ">Ms", where "Ms" is the maximum test result value that the corresponding test system is approved by the government agency to report. In some implementations a minimum value that is less than an actual test result value may be used if that is required by a government agency. In this case, the medical algorithm outputs an alert 109 and a notification 110, which may indicate that the test result value cannot be used because it is outside of an approved reportable range. In report 90, step 102 may be colored or shaded differently than steps 101, 103 (which produced alerts for other reasons) to indicate why that alert was provided. Generating and outputting an alert of this type is an optional feature and need not be included in all reports in all jurisdictions.

In the example of Fig. 15, examples of the following parameters, assays, and predefined values may be the same as those examples described with respect display area 14 of Fig. 9: P1, P2, P3, A1, A2, A4, (AA)mm, (BB)%, (CC)s, (DD), (EE)mm, and (FF)mm. In this example, the test result values may be as follows: Jmm may be 22mm, Ks may be 78s, L% may be 4%; Ms may be 240s, and Nmm may be 7mm. Notification 106 may read "Tranexamic acid 25 mg/kg as a single bolus"; notification 110 may read "Value CT_{IN} can't be used due to being outside the reportable range"; notification 111 may read "Cryoprecipitate or Fibrinogen Concentrate with a target of A5 FIBTEM ≥ 12mm"; notification 112 may read "Platelet concentrate transfusion with a dose of 1-2 pooled or aphresisplatelet concentrates in an adult patient" notification 113 may read "PCC 15-25 IU/kgbw or FFP 10-15 LM/kgbw"; and notification 114 may read "FFP 10-15 LM/kgbw". These treatments and/or diagnoses are as described above.

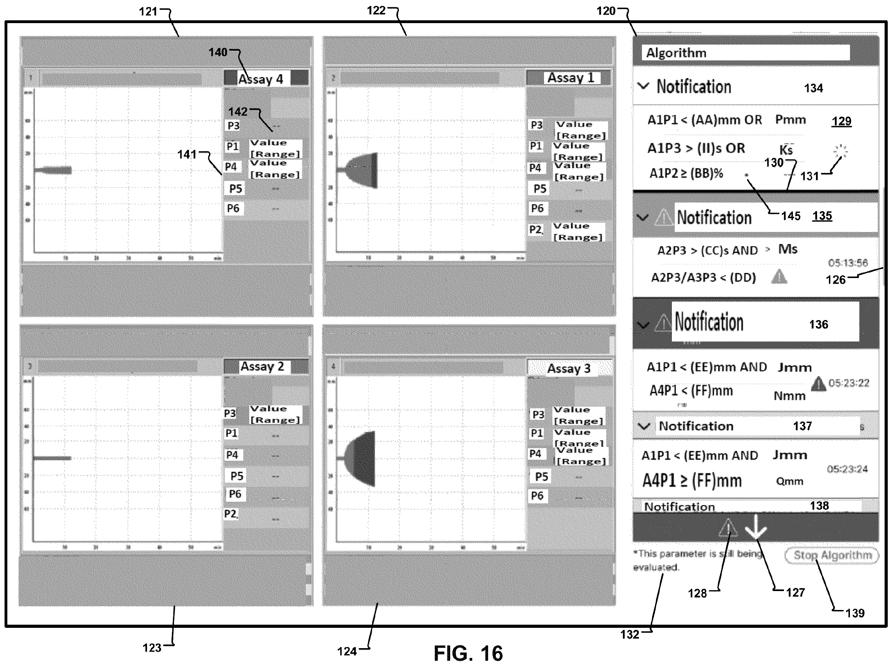

In some implementations, the report is displayed together with the one or more images (described above) from which the test results values to be evaluated by the medical algorithm were obtained. For example, process 85 may retrieve these images from memory and display them with the report. Fig. 16 shows an example implementation where a report 120 is displayed adjacent to such images 121 to 124,

Report 120 has a format, and contains information, similar to report 90 of Fig. 15. Report 120 also contains a scroll bar 126 allows the user to scroll to reach notification boxes not show in the figure. Arrow 127 and corresponding alert 128 indicates that there are one or more alerts that are accessible via the scroll bar. An alert can be displayed if a notification box is collapsed. Button 139 (Stop Algorithm) is a user-selectable button that stops execution of the medical algorithm the algorithm is still processing.

In the example of Fig. 16, a group 129 is separated from other groups in the medical algorithm by a bold line 130. Group 129 displays a progress icon 131 in this example because at least one test result value to be evaluated by group 129 either has not been received or has not been finalized. An asterisk 145 and text 132 identify the test value that has not been received or finalized (for A1P2≥(BB)%).

In the example of Fig. 16, the algorithm is a cardiac algorithm and examples of the following parameters, assays, and values may be the same as those examples described with respect display area 14 of Fig. 9 and report 90 of Fig. 15: P1, P2, P3, A1, A2, A4, (AA)mm, (BB)%, (CC)s, (DD), (EE)mm, (FF)mm, and (Ils). Examples of the following test result values may be the same as those examples described with respect report 90 of Fig. 15: Jmm, Ks, Nmm, and Ms. An example test result value for Pmm may be 42mm and an example test result value for Qmm may be 4mm. Notification box 134 may contain "Tranexamic acid 25 mg/kg as a single bolus"; notification box 135 may contain a statement of "Value CT_{IN} can't be used due to being outside the reportable range"; notification box 136 may contain a statement of "Cryoprecipitate or Fibrinogen Concentrate with a target of A5 FIBTEM ≥ 12mm"; notification box 137 may contain "Platelet concentrate transfusion with a dose of 1-2 pooled or apheresis platelet concentrates in an adult patient"; and notification box 138 may contain a "PCC 15-25 IU/kg bw or FFP 10-15 LM/kg bw".

Each image may identify an assay, such as assay 140, that produced the reaction curve included in the image. Each image may identify parameters, such as parameters 141, that were determined by the corresponding assay. Values and acceptable ranges of values for corresponding parameters are also displayed. For example, for parameters 141, values and ranges 142 are displayed. In an example implementation, the parameters P1, P2, and P3, and assays A1, A2, A3 and A4 may be the same as the examples described with respect display area 14 of Fig. 9. Parameters P4 may be the ROTEM^{®} A10 parameter.

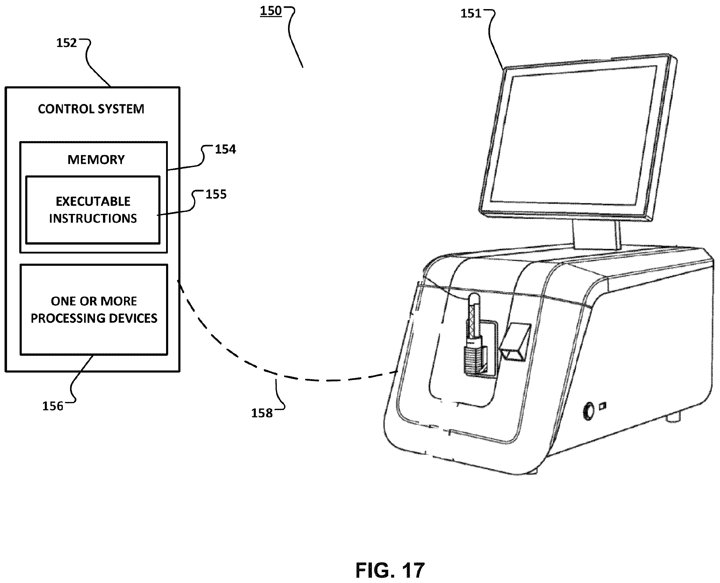

Fig. 17 is a block diagram of test system 150 that includes a test instrument 151 to provide the test results values and a control system 152 to execute process 80 and 85 to build, to verify, and to execute, the medical algorithms described herein. Examples of the test instrument 151 include, but are not limited to, the test instruments identified herein. An example of test instrument 151 is described in U.S. Patent No. 10,175,225 (McCluskey), which issued on January 8, 2019, the contents of which are incorporated herein by reference. Other examples of the test instrument may include, but are not limited to, blood gas analyzers such as the GEM^{®} Premier^{™} 5000 and the GEM^{®} Premier^{™} ChemSTAT^{®} both by Werfen^{®} S.A.

Control system 152 includes machine-readable memory 154 storing instructions 155 that are executable by one or more processing devices 156 to execute processes 80 and 85 and, in some implementations, to control operation of test instrument 151. All or part of the control system may be implemented on a computing system that is external to test instrument 151. All or part of the control system may be internal to test instrument 151. All or part of the control system may be distributed across one or more computing systems and/or the test instrument. Communications between external portions of the control system and the test instrument are represented by dashed line 158.

At least part of the systems and processes described in this specification and their various modifications may be executed or controlled at least in part by one or more computers such as control system 152 using one or more computer programs tangibly embodied in one or more information carriers, such as in one or more non-transitory machine-readable storage media. A computer program can be written in any form of programming language, including compiled or interpreted languages, and it can be deployed in any form, including as a stand-alone program or as a module, part, subroutine, or other unit suitable for use in a computing environment. A computer program can be deployed to be executed on one computer or on multiple computers at one site or distributed across multiple sites and interconnected by a network.

Actions associated with executing the processes or controlling the test system described herein can be performed by one or more programmable processors executing one or more computer programs to control or to perform all or some of the operations described herein. All or part of the test systems and processes can be executed or controlled by special purpose logic circuitry, such as, an FPGA (field programmable gate array) and/or an ASIC (application-specific integrated circuit) or embedded microprocessor(s) localized to the instrument hardware.

Processors suitable for the execution of a computer program include, by way of example, both general and special purpose microprocessors, and any one or more processors of any kind of digital computer. Generally, a processor will receive instructions and data from a read-only storage area or a random-access storage area or both. Elements of a computer include one or more processors for executing instructions and one or more storage area devices for storing instructions and data. Generally, a computer will also include, or be operatively coupled to receive data from, or transfer data to, or both, one or more machine-readable storage media, such as mass storage devices for storing data, such as magnetic, magneto-optical disks, or optical disks. Non-transitory machine-readable storage media suitable for embodying computer program instructions and data include all forms of non-volatile storage area, including by way of example, semiconductor storage area devices, such as EPROM (erasable programmable read-only memory), EEPROM (electrically erasable programmable read-only memory), and flash storage area devices; magnetic disks, such as internal hard disks or removable disks; magneto-optical disks; and CD-ROM (compact disc read-only memory) and DVD-ROM (digital versatile disc read-only memory).

Elements of different implementations described may be combined to form other implementations not specifically set forth previously. Elements may be left out of the systems described previously without adversely affecting their operation or the operation of the system in general. Furthermore, various separate elements may be combined into one or more individual elements to perform the functions described in this specification.

Other implementations not specifically described in this specification are also within the scope of the following claims.

Further aspects, features and embodiments of the present invention are described in the following items:
1. One or more non-transitory machine-readable storage media storing instructions that are executable by one or more processing devices to perform a method comprising:
   outputting a user interface configured to receive inputs to use in a medical algorithm;
   configuring the medical algorithm based on the inputs; and
   outputting a display, the display comprising a representation of the medical algorithm, the representation presenting operations to be executed by the medical algorithm, and the representation relating the operations to one or more notifications triggered based on the operations.
2. The one or more non-transitory machine-readable storage media of item 1,
   wherein the inputs comprise one or more parameters, one or more predefined values, one or more operators, and the one or more notifications;
   wherein the operations comprise one or more rules; and
   wherein a rule is configured to evaluate a test result value for one of the parameters against one of the predefined values based on one of the operators, and
   wherein the notifications correspond to the operations.
3. The one or more non-transitory machine-readable storage media of item 2, wherein the one or more parameters comprise one or more of the following: a parameter associated with viscoelastic testing, a parameter associated with blood gas testing, a parameter associated with platelet function testing, a parameter associated with coagulation testing, or a parameter associated with activated clotting time (ACT) testing.
4. The one or more non-transitory machine-readable storage media of any one of the items 1 to 3, wherein the medical algorithm is for use with a patient; and
   wherein the inputs comprise information about at least one of (i) whether the patient is bleeding or has non-bleeding, thrombosis, (ii) a clinical setting, (iii) a stage of surgery the patient is undergoing, (iv) a medical condition of the patient, (v) a body weight of the patient, (vi) an age of the patient, (vii) a sex of the patient, (viii) a medical diagnosis for the patient, or (ix) a treatment that the patient has undergone or is undergoing.
5. The one or more non-transitory machine-readable storage media of any one of the items 1 to 4, wherein the operations comprise one or more groups;
   wherein a group comprises one or more steps; and
   wherein the group is executable independently of others of the groups.
6. The one or more non-transitory machine-readable storage media of any one of the items 1 to 5, wherein the operations comprise multiple groups, each group comprising multiple steps;
   wherein, within a group among the multiple groups, the steps are executable sequentially; and
   wherein steps in a group among the multiple groups are executable independently of steps in other groups among the multiple groups.
7. The one or more non-transitory machine-readable storage media of any one of the items 1 to 6, wherein the display comprises an element that is selectable to verify the medical algorithm, the element being configured to cause output of the instruction.
8. The one or more non-transitory machine-readable storage media of any one of the items 1 to 7, wherein the inputs comprise parameters, predefined values, and operators;
   wherein the method comprises receiving, from the display, an instruction corresponding to an action to take with respect to the medical algorithm;
   wherein the operations comprise rules configured to evaluate test result values for parameters against respective predefined values based on respective operators; and
   wherein the action comprises verifying the medial algorithm and, in response to the instruction, the method performed by the one or more processing devices comprises outputting a second user interface configured to receive mock or prior test result values for the parameters.
9. The one or more non-transitory machine-readable storage media of item 8, wherein, in response to the instruction, the method performed by the one or more processing devices comprises:
   executing the medical algorithm using the mock or prior test result values received via the second user interface; and
   outputting a report representing one or more results produced by executing the medical algorithm using the or prior mock test result values.
10. The one or more non-transitory machine-readable storage media of item 9, wherein the report comprises one or more of the rules, one or more test result values received via the second user interface for the one or more rules, and at least one of: (i) a time associated with execution of the one or more rules, (ii) a notification triggered by execution of the one or more rules, or (iii) an alert triggered by execution of the one or more rules, the alert being indicative of one or more test result values satisfying the one or more rules.
11. One or more non-transitory machine-readable storage media storing instructions that are executable by one or more processing devices to perform a method comprising:
   receiving test result values for parameters associated with one or more assays; and
   executing a medical algorithm using the test result values, the medical algorithm comprising rules configured to evaluate the test result values, and the medical algorithm comprising groups comprised of one or more steps, with each step comprising one or more of the rules;
   wherein executing the medical algorithm comprises:
      when a group is identified during execution of the medical algorithm, executing the group independently of other groups in the medical algorithm;
      when a series of steps is identified in a group during execution of the medical algorithm, executing the steps in the series in a sequence in which the steps are arranged in the series; and
      outputting a report representing results produced by executing the medical algorithm, wherein the report comprises the rules, values based on test result values associated with corresponding rules, and at least one of: (i) a time associated with execution of the rules, (ii) a notification triggered by execution of the one or more rules, or (iii) an alert triggered by execution one or more of the rules, the alert being indicative of one or more test result values satisfying the one or more rules.
12. The one or more non-transitory machine-readable storage media of item 11, wherein the values based on the test result values comprise at least one of (i) a test result value, (ii) a maximum value that is less than a corresponding test result value, or (iii) a minimum value that is greater than a corresponding test result value.
13. The one or more non-transitory machine-readable storage media of item 11 or 12, wherein the test result values are received from a viscoelastic testing system.
14. The one or more non-transitory machine-readable storage media of any one of the items 11 to 13, wherein the test result values are received in metadata of one or more images or received from an optical character recognition of the one or more images; and
   wherein the method performed by the one or more processing devices further comprises displaying the report together with the one or more images.
15. The one or more non-transitory machine-readable storage media of any one of the items 11 to 14, wherein at least one of the images comprises a reaction curve that shows an elasticity over time when a blood clot forms or dissolves.
16. The one or more non-transitory machine-readable storage media of any one of the items 11 to 15, wherein a test result values for a parameter comprises (i) a final value for the parameter or (ii) a value for the parameter that is non-final.
17. The one or more non-transitory machine-readable storage media of any one of the items 11 to 16, wherein the medical algorithm is for use with a patient;
   wherein the method performed by the one or more processing devices comprises receiving information about at least one of (i) whether the patient is bleeding, (ii) a type of surgery that the patient is undergoing, (iii) a stage of the surgery, or (iv) a medical condition of the patient; and
   wherein the medical algorithm is executed based on the information.
18. A system comprising:
   one or more test instruments configured to produce test result values for parameters associated with one or more assays; and
   one or more processing devices in communication with the one or more test instruments, the one or more processing devices being configured to execute instructions to perform operations comprising:
      receiving the test result values from the one or more test instruments;
      executing a medical algorithm using the test result values, the medical algorithm comprising rules configured to evaluate the test result values, and the medical algorithm comprising groups comprised of one or more steps, with each step comprising one or more of the rules;
      wherein executing the medical algorithm comprises:
         when a group is identified during execution of the medical algorithm, executing the group independently of other groups in the medical algorithm;
         when a series of steps is identified in a group during execution of the medical algorithm, executing the steps in the series in a sequence in which the steps are arranged in the series; and
         outputting a report representing results produced by executing the medical algorithm, wherein the report comprises the rules, values based on test result values associated with corresponding rules, and at least one of: (i) a time associated with execution of the rules, (ii) a notification triggered by execution of the one or more rules, or (iii) an alert triggered by execution one or more of the rules, the alert being indicative of one or more test result values satisfying the one or more rules.
19. The system of item 18, wherein the one or more processing devices are external to the one or more test instruments.
20. The system of item 18, wherein the one or more processing devices are internal to one or more of the test instruments.

## Claims

1. One or more non-transitory machine-readable storage media storing instructions that are executable by one or more processing devices to perform a method comprising:
outputting a user interface configured to receive inputs to use in a medical algorithm;
configuring the medical algorithm based on the inputs; and
outputting a display, the display comprising a representation of the medical algorithm, the representation presenting operations to be executed by the medical algorithm, and the representation relating the operations to one or more notifications triggered based on the operations.

2. The one or more non-transitory machine-readable storage media of claim 1,
wherein the inputs comprise one or more parameters, one or more predefined values, one or more operators, and the one or more notifications;
wherein the operations comprise one or more rules; and
wherein a rule is configured to evaluate a test result value for one of the parameters against one of the predefined values based on one of the operators, and
wherein the notifications correspond to the operations,
wherein preferably the one or more parameters comprise one or more of the following: a parameter associated with viscoelastic testing, a parameter associated with blood gas testing, a parameter associated with platelet function testing, a parameter associated with coagulation testing, or a parameter associated with activated clotting time (ACT) testing.

3. The one or more non-transitory machine-readable storage media of claim 1 or 2, wherein the medical algorithm is for use with a patient; and
wherein the inputs comprise information about at least one of (i) whether the patient is bleeding or has non-bleeding, thrombosis, (ii) a clinical setting, (iii) a stage of surgery the patient is undergoing, (iv) a medical condition of the patient, (v) a body weight of the patient, (vi) an age of the patient, (vii) a sex of the patient, (viii) a medical diagnosis for the patient, or (ix) a treatment that the patient has undergone or is undergoing.

4. The one or more non-transitory machine-readable storage media of any one of the claims 1 to 3, wherein the operations comprise one or more groups;
wherein a group comprises one or more steps; and
wherein the group is executable independently of others of the groups.

5. The one or more non-transitory machine-readable storage media of any one of the claims 1 to 4, wherein the operations comprise multiple groups, each group comprising multiple steps;
wherein, within a group among the multiple groups, the steps are executable sequentially; and
wherein steps in a group among the multiple groups are executable independently of steps in other groups among the multiple groups.

6. The one or more non-transitory machine-readable storage media of any one of the claims 1 to 5, wherein the display comprises an element that is selectable to verify the medical algorithm, the element being configured to cause output of the instruction.

7. The one or more non-transitory machine-readable storage media of any one of the claims 1 to 6, wherein the inputs comprise parameters, predefined values, and operators;
wherein the method comprises receiving, from the display, an instruction corresponding to an action to take with respect to the medical algorithm;
wherein the operations comprise rules configured to evaluate test result values for parameters against respective predefined values based on respective operators; and
wherein the action comprises verifying the medical algorithm and, in response to the instruction, the method performed by the one or more processing devices comprises outputting a second user interface configured to receive mock or prior test result values for the parameters,
wherein preferably, in response to the instruction, the method performed by the one or more processing devices comprises:
executing the medical algorithm using the mock or prior test result values received via the second user interface; and
outputting a report representing one or more results produced by executing the medical algorithm using the mock or prior test result values.

8. The one or more non-transitory machine-readable storage media of claim 7, wherein the report comprises one or more of the rules, one or more test result values received via the second user interface for the one or more rules, and at least one of: (i) a time associated with execution of the one or more rules, (ii) a notification triggered by execution of the one or more rules, or (iii) an alert triggered by execution of the one or more rules, the alert being indicative of one or more test result values satisfying the one or more rules.

9. One or more non-transitory machine-readable storage media storing instructions that are executable by one or more processing devices to perform a method comprising:
receiving test result values for parameters associated with one or more assays; and
executing a medical algorithm using the test result values, the medical algorithm comprising rules configured to evaluate the test result values, and the medical algorithm comprising groups comprised of one or more steps, with each step comprising one or more of the rules;
wherein executing the medical algorithm comprises:
when a group is identified during execution of the medical algorithm, executing the group independently of other groups in the medical algorithm;
when a series of steps is identified in a group during execution of the medical algorithm, executing the steps in the series in a sequence in which the steps are arranged in the series; and
outputting a report representing results produced by executing the medical algorithm, wherein the report comprises the rules, values based on test result values associated with corresponding rules, and at least one of: (i) a time associated with execution of the rules, (ii) a notification triggered by execution of the one or more rules, or (iii) an alert triggered by execution of one or more of the rules, the alert being indicative of one or more test result values satisfying the one or more rules.

10. The one or more non-transitory machine-readable storage media of claim 9, wherein the values based on the test result values comprise at least one of (i) a test result value, (ii) a maximum value that is less than a corresponding test result value, or (iii) a minimum value that is greater than a corresponding test result value.

11. The one or more non-transitory machine-readable storage media of claim 9 or 10, wherein the test result values are received from a viscoelastic testing system, and/or
wherein the test result values are received in metadata of one or more images or received from an optical character recognition of the one or more images; and
wherein the method performed by the one or more processing devices further comprises displaying the report together with the one or more images.

12. The one or more non-transitory machine-readable storage media of any one of the claims 9 to 11, wherein at least one of the images comprises a reaction curve that shows an elasticity over time when a blood clot forms or dissolves, and/or
wherein a test result values for a parameter comprises (i) a final value for the parameter or (ii) a value for the parameter that is non-final.

13. The one or more non-transitory machine-readable storage media of any one of the claims 9 to 12, wherein the medical algorithm is for use with a patient;
wherein the method performed by the one or more processing devices comprises receiving information about at least one of (i) whether the patient is bleeding, (ii) a type of surgery that the patient is undergoing, (iii) a stage of the surgery, or (iv) a medical condition of the patient; and
wherein the medical algorithm is executed based on the information.

14. A system comprising:
one or more test instruments configured to produce test result values for parameters associated with one or more assays; and
one or more processing devices in communication with the one or more test instruments, the one or more processing devices being configured to execute instructions to perform operations comprising:
receiving the test result values from the one or more test instruments;
executing a medical algorithm using the test result values, the medical algorithm comprising rules configured to evaluate the test result values, and the medical algorithm comprising groups comprised of one or more steps, with each step comprising one or more of the rules;
wherein executing the medical algorithm comprises:
when a group is identified during execution of the medical algorithm, executing the group independently of other groups in the medical algorithm;
when a series of steps is identified in a group during execution of the medical algorithm, executing the steps in the series in a sequence in which the steps are arranged in the series; and
outputting a report representing results produced by executing the medical algorithm, wherein the report comprises the rules, values based on test result values associated with corresponding rules, and at least one of: (i) a time associated with execution of the rules, (ii) a notification triggered by execution of the one or more rules, or (iii) an alert triggered by execution of one or more of the rules, the alert being indicative of one or more test result values satisfying the one or more rules.

15. The system of claim 14, wherein the one or more processing devices are external to the one or more test instruments, or
wherein the one or more processing devices are internal to one or more of the test instruments.
